# EUROPÄISCHE PATENTSCHRIFT

(11) **EP 0 717 111 B1**
(45) Veröffentlichungstag und Bekanntmachung des Hinweises auf die Patenterteilung: **26.08.1998**
(21) Anmeldenummer: 95119232.7
(22) Anmeldetag: 06.12.1995
(51) Int. Cl.: C12P 7/26

(54) **Mikrobilles Verfahren zur Herstellung von Dihydroxyaceton unter Rückführung von Biomasse**
Microbial process for the preparation of dihydroxyacetone with recirculation of biomass
Procédé microbien pour la production de dihydroxyacétone avec recirculation de la biomasse

(30) Priorität: 14.12.1994 DE 4444404
(43) Veröffentlichungstag der Anmeldung: 19.06.1996
(73) Patentinhaber: MERCK PATENT GmbH, 64293 Darmstadt (DE)
(72) Erfinder: Ohrem, Hans L., Dr., D-64331 Weiterstadt (DE); Westermeier, Frank, Dr., D-64839 Münster (DE)

(56) Entgegenhaltungen:
- US-A- 1 833 716
- CHEMICAL ABSTRACTS, vol. 96, no. 5, 1.Februar 1982 Columbus, Ohio, US; abstract no. 33389u, MIRONOVA, T.N. ET AL. 'Dihydroxyacetone' Seite 508; Spalte l;
- CHEMICAL ABSTRACTS, vol. 81, no. 7, 19.August 1974 Columbus, Ohio, US; abstract no. 36469, POMORTSEVA, N.V. ET AL. 'Preparation of dihydroxyacetone using glycerol oxidation by a suspension of Acetobacter suboxydans quiescent cells' Seite 307; Spalte r;
- CURRENT MICROBIOLOGY, Bd. 25, Nr. 3, September 1992 Seiten 149-155, XP 000566648 C. CLARET ET AL. 'Glycerol inhibition of growth and dihydroxyacetone production by Gluconobacter oxydans'
- DATABASE WPI Section Ch, Week 9550 Derwent Publications Ltd., London, GB; Class D16, AN 95-391458 'Di:hydroxy-acetone production by continuous bio-transformation process of glycerol' & RU-A-2 031 123 (BARBOT V.S. ET AL.) , 20.März 1995
- BIOFORUM, Bd. 18, Oktober 1995 Seiten 358-363, XP 000566656 H.L. OHREM ET AL. 'Optimierung einer industriellen Fermentation durch ein prozeszkinetisches Modell'

## Beschreibung

Die Erfindung betrifft ein mikrobielles Verfahren zur Herstellung von Dihydroxyaceton, wobei die Umsetzung von Glycerin zu Dihydroxyaceton mit Hilfe eines dehydrogenaseaktiven Mikroorganismus unter periodischer teilweiser Rückführung von nicht mehr wachstumsfähiger Biomasse und entsprechend den Produktionszyklen abgestimmter Zugabe von voll wachstumsfähigen Vorkulturen durchgeführt wird.

Die Gewinnung von Dihydroxyaceton (DHA) aus Glycerin mit geeigneten Mikroorganismen ist seit langem allgemein bekannt. Da Dihydroxyaceton ein wertvolles Ausgangsprodukt für die chemische und pharmazeutische Industrie darstellt, besteht ein permanentes Interesse, die Verbindung auf möglichst einfache und wirtschaftliche Weise im großtechnischen Maßstab zur Verfügung zu stellen. Entsprechend wurden im Laufe der Jahre Variationen an den Kultivierungs - und Fermentationsbedingungen vorgenommen mit dem Ziel, ständig die Ausbeute zu erhöhen (z.B. US 2,948,658, US 4,076,589, DD 230 713, DD 253 836).

Aus der US 1,833,716 ist zudem ein über mehrere Produktionszyklen laufendes Verfahren bekannt, in dem die Biomasse durch wiederholtes Zufügen von Produktionsmedium immer wieder verwendet wird.

Die Fermentationsbrühen des Standes der Technik enthalten Glycerin zumeist in 5-20% iger Konzentration, das in einigen wenigen Fällen zugefüttert wird. Zusatzstoffe wie Glucose, Sorbit, Hefe-Extrakte, Maisquellwasser und bei der Bakterienkultivierung übliche Salze sind in Konzentrationen in der Regel zwischen unter 1% und bis zu 15% im Fermentationsansatz enthalten und der pH-Wert während der mikrobiellen Synthese von Dihydroxyaceton variiert in der Regel zwischen 5 und 7. Die im Stand der Technik angegebenen Ausbeuten an Dihydroxyaceton variieren durchschnittlich zwischen 10 und 150 g/l bei einer Kultivierungs- bzw. Reaktionszeit von 20 bis 70 h in Abhängigkeit vom Fermentationstyp (Batchverfahren oder vollkontinuierliche Fermentation).

Bei der mikrobiellen DHA-Synthese des Standes der Technik wurde insbesondere der Tatsache Aufmerksamkeit geschenkt, daß sowohl der Menge an Edukt (Glycerin) als auch an synthetisiertem Produkt (Dihydroxyaceton) offensichtlich eine entscheidende Rolle beigemessen werden muß, da beide Verbindungen, wenn sie in größeren Konzentrationen in der Fermentationsbrühe vorliegen, Wachstum und/oder Produktivität des eingesetzten Mikroorganismus in negativer Weise beeinflussen (z.B. Claret et al. (1992), Current Microbiol. 25(3), 149). So erleiden die Zellen der verwendeten Kultur im Laufe des Produktionsvorganges als Folge des sich anreichernden Dihydroxyacetons irreversible Schädigungen, so daß sie nach einer Produktionsdauer von etwa 20 bis 30 Stunden nicht mehr oder nur sehr begrenzt vermehrungsfähig sind und aus dem eigentlichen Produktionsvorgang ausgeschleust werden müssen. Dies hat zur Konsequenz, daß für den nächsten Produktionszyklus eine vollständig neue Kultur herangezogen werden muß. Entsprechende Vorkulturen müssen dann in der Regel in den Produktionsfermenter überführt werden, in dem die Kultur wiederum eine begrenzte Zeit wachsen soll. Das Wachstum in der Produktionsstufe ist zur Erzielung einer zufriedenstellenden Produktivität erforderlich. Daher müssen auch in diesem Maßstab Nährsalze und komplexe Stickstoffquellen, wie z. B. Hefeextrakt, in sterilisierter Form vorgelegt werden. Dies verursacht einen großen Teil der Rohstoffkosten, sowie Probleme bei der Aufbereitung, Isolierung und Qualitätssicherung des Endproduktes. Überdies ist bei dieser üblichen Verfahrensweise die Raum-Zeit-Ausbeute des Produktes (in g/l x h ) des gesamten Systems eng an die Wachstumsgeschwindigkeit im Produktionsfermenter gekoppelt, welche wiederum, wie oben erwähnt, durch das entstehende Dihydroxyaceton begrenzt wird. Außerdem stellen nicht produktive Vorkulturzeiten einen nicht unerheblichen Anteil der benötigten Gesamtprozesszeit dar.

Eine Entkopplung von Wachstum und eigentlicher Produktion wäre somit wünschenswert.

Es wurde nun festgestellt, daß einerseits dehydrogenaseaktive Mikroorganismen bei Kultivierung in Abwesenheit ihres eigentlichen Substrates Glycerin und in Anwesenheit von Monosacchariden oder Zuckeralkoholen über einen längeren Zeitraum hinweg ihre Vermehrungsfähigkeit nicht verlieren, andrerseits nach Zusetzen von Glycerin und der entsprechend der enzymatischen Reaktion fortschreitenden Anreicherung von DHA zwar die ihre Wachsumsfähigkeit, nicht jedoch ihre eigentliche katalytische Aktivität verlieren.

Es wurde nun gefunden, daß die genannten Erkenntnisse als Grundlage für eine neue und vorteilhafte auch im großtechnischen Maßstab anwendbare Herstellung von DHA mittels Mikroorganismen im Batchverfahren dienen können. Danach wird die nicht mehr wachstumsfähge Biomasse nach einem durchlaufenen Produktionszyklus nicht mehr aus dem Produktionsprozess entfernt sondem verbleibt vollständig oder zumindest teilweise im Fermenter und dient dort als Vorlage für den folgenden DHA-Produktionszyklus.

Gegenstand der Erfindung ist somit Verfahren zur Herstellung von Dihydroxyaceton durch Dehydrierung von Glycerin mittels dehydrogenaseaktiver Mikroorganismen in mehreren Produktionszyklen, daß dadurch charakterisiert werden kann, daß die nach einem Produktionzyklus erhaltene, nicht mehr wachstumsfähige Biomasse ganz oder teilweise für den jeweils folgenden Produktionszyklus wiederverwendet wird.

Falls Teile, vorzugsweise 10 bis 30%, der vermehrungsunfähigen Biomasse nach einem Zyklus entfernt werden, wird mit frischer wachstumsfähiger Vorkultur ergänzt. Erfindungsgemäß wird hierbei die zuzuführende Kultur in einem glycerinfreien Medium, in dem sich somit keine zellschädigende DHA anreichem kann, angezüchtet.

Gegenstand der Erfindung ist somit auch ein dementsprechendes Verfahren, in dem dem folgenden oder einem diesen Zyklus folgenden Produktionszyklus eine wachstumsfähige Vorkultur, die vorzugsweise in einem glycerinfreien Medium angezüchtet wurde und 10 bis 20 % der Gesamtbiomasse ausmacht, zugesetzt.

Gegenstand der Erfindung ist insbesondere ein Verfahren, bei dem die Zugabe der wachstumsfähigen Vorkultur zu einem der folgenden Produktionszyklen in der Weise erfolgt, daß die Zugabe der Vorkultur entweder nach unmittelbar jedem oder nach jedem zweiten bis fünften Zyklus erfolgt, wobei die Steuerung der Zugabe der Vorkultur in der Weise erfolgen sollte, daß die Ausbeute an Dihydroxyaceton in allen Fällen nicht unter einen Wert von 4 g/l h sinkt.

Erfindungsgemäß eignen sich alle jenen bekannten Mikroorganismen, die aufgrund aktiver Dehydrogenasen zu oxidieren vermögen und Glycerin als Substrat erkennen. Vorallem die Gattungen *Acetobacter* und *Gluconobacter* sind hierfür einsetzbar, insbesondere *Gluconobacter.* Unter den Gluconobacter-Arten ist vorzugsweise *Gluconobacter oxydans* geeignet. Beispiele für allgemein zugängliche *Gluco*nobacter *oxydans* Stämme sind die Stämme ATCC 621(DSM50049), DSM2343, ATCC 9324, ATCC23771 und DSM2003.

Die benötigten wachstumsfähigen Vorkulturen werden vorzugsweise in einem glycerinfreien Kulturmedium gezüchtet. Als Kohlenstoffquelle dienen Monosaccharide oder Zuckeralkohole, insbesondere Sorbit, Mannit, Fructose oder Glucose, wobei die Zuckeralkohole, insbesondere Sorbit, bevorzugt sind. Auf Glycerin als Energie- und Kohlenstoffquelle wird bewußt verzichtet, um keine DHA Bildung in dieser reinen Wachstumsphase zu erhalten. Der Gehalt an Zuckeralkoholen in dem Kulturmedium liegt erfindungsgemäß zwischen 50 und 300 g/l vorzugsweise zwischen 100 und 150 g/l.

Das Kulturmedium für die Vorkultur enthält vorzugsweise neben den üblichen für derartige Zwecke bekannten Nährsalzen als komplexe Stickstoffquelle einen Hefeextrakt. Jedoch sind auch andere bekannte Stickstoffquellen, wie Aminosäuren oder Maisquellwasser für das erfindungsgemäße Verfahren oder auch Hefeextrakte, die mit Aminosäuren angereichert wurden, einsetzbar. Der Gehalt an Hefeextrakt bzw. Aminosäuren liegt erfindungsgemäß zwischen 1 und 10 g/l, vorzugsweise zwischen 3 und 6 g/l.

Die Biomasse der Vorkultur wird in den Produktionsfermenter überführt und für den ersten Produktionszyklus mit Nährmedium versetzt. Das Nährmedium enthält die üblichen für diese Zwecke geeigneten Salze, sowie entsprechende, wie bereits bei der Kultivierung der Vorkultur verwendeten Stickstoffquellen, insbesondere Hefeextrakte. Zusätzlich wird nun das Substrat hinzugegeben. Der Ausgangsgehalt an Glycerin liegt zwischen 20 und 100 g/l, vorzugsweise zwischen 30 und 60 g/l, in einer besondersbevorzugten Ausführungsform bei 50 g/l, Während eines Produktionszyklus, der erfindungsgemäß zwischen 10 und 30, vorzugsweise aber zwischen 15 und 25 Stunden dauert, wird der Glyceringehalt vorzugsweise durch stetige Nachdosierung auf einer Konzentration von 2 bis 50 g/l, vorzugsweise von 5 bis 35 g/l, gehalten. Der jeweilige Glyceringehalt in der Reaktionssuspension wird nach allgemein bekannten Methoden bestimmt, beispielsweise durch Probenentnahme und Glycerinbestimmung mittels chromatographischer Bestimmungen. Das Einstellen eines weitgehend konstanten Glyceringehaltes hat sich als sehr positiv für die Raum-Zeit-Ausbeute erwiesen, ist aber letzlich in diesem Zusammenhang nicht unbedingt erfindungswesentlich. Der pH-Wert wird während der Fermentation vorzugsweise konstant bei einem bestimmten pH-Wert zwischen 3.8 und 4.8 gehalten. Vorzugsweise wird ein pH-Wert von 4.0 bis 4.5 eingestellt. Die Einstellung erfolgt vorzugsweise mit Calciumhydroxid-Lösung. Überhaupt hat der Einsatz von Calciumionen, beispielsweise in Form von Calciumchlorid einen günstigen Einfluß auf die Ausbeute. Der Gehalt an Calciumionen liegt zwischen 1 g/l und etwa 5 g/l. Die Fermentationstemperatur liegt erfindungsgemäß zwischen 28 und 34°C, vorzugsweise zwischen 30 und 32°C. Die Fermentationsbrühe wird in üblicher Weise mit Luft oder Sauerstoff belüftet.

In der ersten Variante des Verfahrens wird nach dem ersten Produktionszyklus die gesamte abgetrennte Biomasse, die in der Regel nicht mehr vermehrungsfähig ist, nicht vemichtet, sondem in den Produktionszyklus vollständig zurückgeführt. Der Fermenter wird in diesen Fällen nicht mit Kulturmedium sondem ausschließlich mit Calciumionen-haltiger Substratlösung (z. B.: 1 g/l bis ca. 5 g/l Calciumchlorid und 5 g/l bis 200 g/l Glycerin) befüllt, die nicht sterilisiert zu werden braucht. Der Glyceringehalt wird auch hier, wie oben beschrieben, durch Zufütterung konstant gehalten. Auch bei den folgenden Zyklen kann man analog vorgehen.Da auf diese Weise im Dauerbetrieb die Biomasse von Zyklus zu Zyklus steigt, empfiehlt es sich, einen Teil der Biomasse auszuschleusen. Bei dieser Verfahrensvariante verzichtet man bewußt auf einen Teil der spezifischen Produktivität (Produktbildung pro Zeiteinheit), kann aber die Raum-Zeit-Ausbeute durch Erhöhung der Biomasse auf fast jeden beliebigen Wert einstellen, wobei allerdings die maximale Sauerstoffübertragungsrate des Belüftungssystems eine technisch-bedingte Obergrenze darstellt.Nach etwa fünf Zyklen stellt man in der Regel einen anfangs langsamen, später rascher eintretenden Verlust an katalytischer Aktivität (Umsetzung von Glycerin zu DHA) fest. Erfindungsgemäß wird daher etwa nach jedem dritten Produktionszyklus ein Teil der Biomasse, vorzugsweise 10 bis 30 %, insbesondere 10 bis 20 %, durch voll wachstumsfähige frische Vorkultur ersetzt. Hierbei empfiehlt sich dem Fermenter wieder ganz oder teilweise Kulturmedium zuzuführen, da dies zu leicht verbesserten Ausbeuten führt. Aus verfahrensökonomischen Gründen kann aber auch hier zumindest für einige Produktionszyklen ausschließlich Calciumionen-haltige Substratlösung verwendet werden. Die Zugabe der wachstumsfähigen Vorkultur nach bestimmten Produktionszyklen sollte zweckmäßigerweise immer dann getätigt werden, wenn die Ausbeute an DHA unter einen Wert von 4 g/l h zu sinken droht. Bei Fermentationen unter diesem Wert für einen längeren Zeitraum ist die Wirtschaftllichkeit des Verfahrens nur noch begrenzt gegeben.

In einer zweiten Verfahrensvariante erfolgt die Zugabe der frischen Vorkultur prinzipiell nach jedem oder zumindest nach jedem zweiten Zyklus. Diese Variante produziert die höchsten Ausbeuten an DHA verbunden mit einer noch vorteilhaften und gegenüber dem Stand der Technik gesteigerten Verfahrensökonomie, während die Verfahrensvariante 1 mehr Wert auf die Verfahrensökonomie legt, wobei die Ausbeuten hier etwas unter den maximal zu erreichenden liegen.

Das gebildete Dihydroxyaceton kann aus der Fermenterlösung nach Standardmethoden isoliert und aufgereinigt werden.

Nach dem erfindungsgemäßen Verfahren, können DHA-Ausbeuten von 8 bis 14 g/l x h bzw. 160 bis 250 g/l x Zyklus (1 Zyklus = ca. 20 Stunden) erreicht werden.

Die Vorteile des erfindungsgemäßen verfahrens gegenüber den bekannten Batchverfahren des Standes der Technik sind :
- geringere Rohstoffkosten,
- Zeit- und Energieeinsparung bei Sterilisation und Vorbereitung des Fermenters,
- Erleichterte und quantitatv verbesserte Aufarbeitung des Produkts, z. B. höhere Membranfilter-Flußleistungen (Filtration der mit Produkt angereicherten Lösung),
- weniger Qualitätsschwankungen durch weniger Begleitstoffe (bedingt durch die geringeren Mengen an benötigten Medien),
- geringere Belastung des Produktes mit Sulfatasche (aus Medien, entspricht der Summe der anorganischen Salze),
- innerhalb weiter Grenzen freie Einstellbarkeit der Raum-Zeit-Ausbeute,
- Abfallvermeidung und geringere Belastung der Kläranlage.

### Beschreibung der Abbildungen:

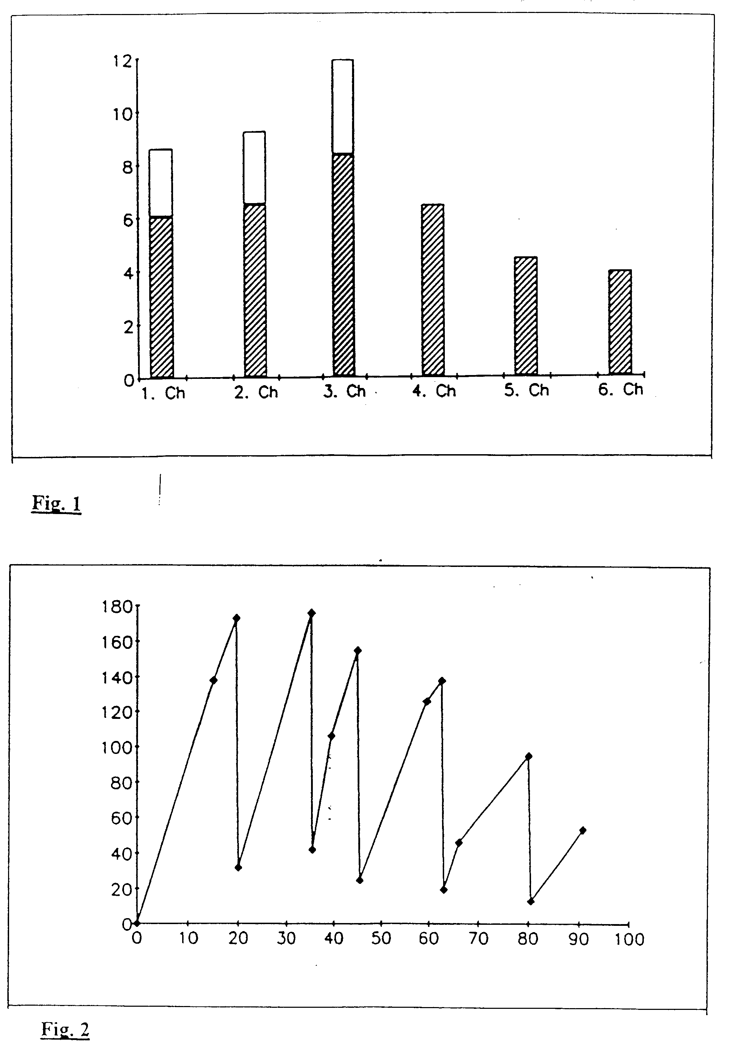

Fig. 1:
   Raum-Zeit-Ausbeute als Maß für die Produktivität bei DHA-Fermentationen nach Beispiel 1 (1. bis 3. Produktionszyklus) und nach Beispiel 2 (3. bis 6. Produktionszyklus). Durch Zufuhr frischer Vorkulturen kann die Produktivität gesteigert oder gehalten werden. Ohne Zufuhr der Vorkultur verliert die Biomasse anfangs relativ viel später nur noch wenig Produktivität. Auf der x-Achse sind die Chargen-Nummern (Produktionszyklen), auf der y-Achse die Raum-Zeit-Ausbeuten in g/l h DHA angegeben. Die schraffierten Bereiche geben den permanenten Anteil die weißen Bereiche den wachstumsassozierten Anteil wieder.
Fig. 2:
   Verlauf der DHA-Konzentration in den sechs Produktionszyklen nach Fig. 1. Auf der x-Achse ist die Fermentationszeit (h) und auf der y-Achse die DHA-Konzentration (g/l) angegeben.

### Beispiel 1:

Zur Umsetzung von Glycerin zu DHA werden 100 1 eines Mediums mit folgender Zusammensetzung angesetzt:

| | |
|---|---|
| Sorbit | 150 g / l |
| Hefeextrakt | 5 g / l |
| Ammmoniumsulfat | 2 g / l |

Die Lösung wird sterilisiert (20 min, 120 °C) und nach dem Abkühlen mit 5 bis 10 l einer dicht bewachsenen Vorkultur des Bakteriums *Gluconobacter oxydans* (ATCC 621; 2.5 g Trockenmasse/l) beimpft. Bei guter Belüftung (50 l/min) und starker Durchmischung läßt man die Bakterienkultur ca. 10 bis 15 h lang wachsen. Die so gewonnene Biomasse wird als Vorkultur in einen 1000 l Fermenter überführt, der 700 l bereits sterilisiertes Nährmedium mit folgender Zusammensetzung enthält:

| | |
|---|---|
| Glycerin | 50 g / l |
| Hefeextrakt | 5 g / l |
| Ammoniumsulfat | 2 g / l |

Unter starker Belüftung (300 l/min) und Durchmischung wird der Glyceringehalt durch stetige Nachdosierung auf einer Konzentration von 25 bis 35 g/l gehalten. In dieser Weise wird für 10 bis 25 Stunden Glycerin zu DHA oxidiert.

Nach Abbruch der Fermentation wird die im Fermenter befindliche Biomasse durch Mikrofiltration (0.2 µm) abgetrennt und 90 % davon in den Fermenter zurückgeführt. Das Filtrat wird einer weiteren Produktaufbereitung zugeleitet. Der Fermenter wird nun mit Calciumchlorid-Substratlösung (3 g/l Calciumchlorid und 50 g/l Glycerin), welche zuvor nicht sterilisiert wurde, bis auf ein Volumen von 700 l befüllt. Nachdem eine erneut hergestellte Vorkultursuspension (in Nährmedium) in den Fermenter überführt wurde, wird der nächste Produktionszyklus gestartet. Die beschriebene Verfahrensweise wiederholt sich nach jedem Produktionszyklus. Nach dem ersten Zyklus werden 180 g/l, nach dem zweiten Zyklus 200 g/l und nach dem dritten 210 g/l DHA gewonnen (Fig. 1).

### Beispiel 2:

Ein Produktionszyklus wird analog Beispiel 1 durchgeführt. Im folgenden Produktionszyklus wird der Fermenter nicht mit Nährlösung, sondem mit 10 % - iger Glycerin-Lösung und 3 % - iger Calciumchlorid-Lösung bis 800 l aufgefüllt. Zusätzlich werden 100 % der Biomasse aus dem vorangegangen Produktionszyklus zugeführt. Durch Nachdosierung wird der Glyceringehalt ebenso auf 5 bis 35 g/l gehalten. In dieser Art können drei bis fünf Produktionszyklen ohne Zufuhr von Nährlösung und Vorkulturen durchgeführt werden. Durch abnehmende Produktivität wird der Fermenter spätestens nach dem fünften Produktionszyklus mit frischer Vorkultur neu gestartet (Fig. 2).

### Beispiel 3:

Ein Produktionszyklus wird analog Beispiel 1 durchgeführt. Für den nachfolgenden Zyklus wird der Fermenter nun nicht mit Calcium-Substratlösung sondem mit Nährmedium beschickt. Die Zugabe von Frisch kultur erfolgt nach jedem weiteren Zyklus. Nach dem ersten Zyklus werden 190 g/l, nach dem zweiten Zyklus 220 g/l und nach dem dritten 235 g/l DHA gewonnen.

## Patentansprüche

1. Verfahren zur Herstellung von Dihydroxyaceton durch Dehydrierung von Glycerin mittels dehydrogenaseaktiver Mikroorganismen in mehreren Produktionszyklen, dadurch gekennzeichnet, daß die Mikroorganismen in Abwesenheit von Glycerin und in Anwesenheit von Monosacchariden oder Zuckeralkoholen zunächst kultiviert werden und die nach einem Produktionzyklus in Anwesenheit von Glycerin erhaltene, nicht mehr wachstumsfähige Biomasse lediglich teilweise für den jeweils folgenden Produktionszyklus wiederverwendet wird.

2. Verfahren nach Anspruch 1, dadurch gekennzeichnet, daß 70 bis 90 % der Biomasse wiederverwendet werden.

3. Verfahren nach Anspruch 1 oder 2, dadurch gekennzeichnet, daß der folgende Produktionszyklus ohne Zugabe von zusätzlichem Wachstums-medium in Anwesenheit von wäßriger Calciumionen-haltiger Lösung und einem pH-Wert von 4.0 bis 5.5 durchgeführt wird.

4. Verfahren nach Anspruch 3, dadurch gekennzeichnet, daß dem folgenden oder einem diesen Zyklus folgenden Produktionszyklus eine wachstums-fähige Vorkultur zugesetzt wird.

5. Verfahren nach Anspruch 4, dadurch gekennzeichnet, daß die Vorkultur in einem glycerinfreien Medium angezüchtet wurde.

6. Verfahren nach Anspruch 5, dadurch gekennzeichnet, daß die Vorkultur in einem Zuckeralkohol-haltigen Medium angezüchtet wurde.

7. Verfahren nach Anspruch 4, 5 oder 6, dadurch gekennzeichnet, daß die Biomasse der zugesetzten Vorkultur 10 - 20 % der Gesamtbiomasse ausmacht.

8. Verfahren nach einem der Ansprüche 4 bis 7, dadurch gekennzeichnet, daß Produktionszyklen mit Zugabe von wachstumsfähiger Vorkultur entsprechende Produktionszyklen ohne Zugabe von Vorkultur folgen.

9. Verfahren nach Anspruch 8 dadurch gekennzeichnet, daß die Zugabe der wachstumsfähigen Vorkultur zu einem der folgenden Produktionszyklen nach jedem dritten bis fünften Zyklus erfolgt.

10. Verfahren nach einem der Ansprüche 1 bis 9, dadurch gekennzeichnet, daß als dehydrogenaseaktiver Mikroorganismus *Gluconobacter oxydans* eingesetzt wird.

11. Verfahren zur Herstellung von Dihydroxyaceton durch Dehydrierung von Glycerin mittels dehydrogenaseaktiver Mikroorganismen, welche zuvor in Abwesenheit von Glycerin und in Anwesenheit von Monosacchariden oder Zuckeralkoholen kultiviert wurden, in mehreren Produktionszyklen, wobei die nach einem Produktionzyklus erhaltene, nicht mehr wachstumsfähige Biomasse vollständig für den jeweils folgenden Produktionszyklus wiederverwendet wird, dadurch gekennzeichnet, daß der auf einen Produktionszyklus folgende Zyklus ohne Zugabe von zusätzlichem Wachstumsmedium in Anwesenheit von wäßriger Calciumionen-haltiger Lösung bei einem pH-Wert von 4.0 bis 5.5 durchgeführt wird.

## Claims

1. Process for the preparation of dihydroxyacetone by dehydrogenation of glycerol by means of microorganisms having active dehydrogenase in a plurality of production cycles, characterized in that the microorganisms are first cultured in the absence of glycerol and in the presence of monosaccharides or sugar alcohols and the biomass which is no longer capable of growth and which is obtained after one production cycle in the absence of glycerol is reused only in part for the respective following production cycle.

2. Process according to Claim 1, characterized in that 70 to 90 % of the biomass is reused.

3. Process according to Claim 1 or 2, characterized in that the subsequent production cycle is performed without addition of additional growth medium and in the presence of aqueous calcium-ion-containing solution and at a pH of 4.0 to 5.5.

4. Process according to Claim 3, characterized in that a preliminary culture capable of growth is added to the subsequent production cycle or a production cycle following this cycle.

5. Process according to Claim 4, characterized in that the preliminary culture was grown in a glycerol-free medium.

6. Process according to Claim 5, characterized in that the preliminary culture was grown in a sugar-alcohol-containing medium.

7. Process according to Claim 4, 5 or 6, characterized in that the biomass of the added preliminary culture makes up 10 - 20 % of the total biomass.

8. Process according to one of Claims 4 to 7, characterized in that production cycles with addition of preliminary culture capable of growth follow corresponding production cycles without addition of preliminary culture.

9. Process according to Claim 8, characterized in that the preliminary culture capable of growth is added to one of the subsequent production cycles after every third to fifth cycle.

10. Process according to one of Claims 1 to 9, characterized in that the microorganism having active dehydrogenase used is *Gluconobacter* oxydans.

11. Process for the preparation of dihydroxyacetone by dehydrogenation of glycerol by means of microorganisms having active dehydrogenase which have been cultured in advance in the absence of glycerol and in the presence of monosaccharides or sugar alcohols in a plurality of production cycles, the biomass which is no longer capable of growth and which is obtained after one production cycle being completely reused for the respective following production cycle, characterized in that the cycle following a production cycle is performed without addition of additional growth medium in the presence of aqueous calcium-ion-containing solution at a pH of 4.0 to 5.5.

## Revendications

1. Procédé pour la préparation de la dihydroxyacétone par deshydrogénation du glycérol à l'aide de microorganismes ayant une activité de deshydrogénase en plusieurs cycles de production, caractérisé en ce que les microorganismes sont d'abord cultivés en l'absence de glycérol et en présence de monosaccharides ou d'alcools des sucres et la biomasse obtenue après un cycle de production en présence de glycérol, qui n'est plus aptes à la croissance, n'est réutilisée qu'en partie pour le cycle de production suivant.

2. Procédé selon la revendication 1, caractérisé en ce que l'on réutilise de 70 à 90 % de la biomasse.

3. Procédé selon la revendication 1 ou 2, caractérisé en ce que le cycle de production suivant est réalisé sans adjonction de compléments du milieu de croissance, en présence d'une solution aqueuse contenant des ions calcium et à un pH de 4,0 à 5,5.

4. Procédé selon la revendication 3, caractérisé en ce que, au cycle suivant ou à un cycle de production consécutif, on ajoute une culture préalable apte à la croissancee.

5. Procédé selon la revendication 4, caractérisé en ce que la culture préalable est réalisée dans un milieu exempt de glycérol.

6. Procédé selon la revendication 5, caractérisé en ce que la culture préalable est réalisé dans un milieu contenant des alcools de sucres.

7. Procédé selon la revendication 4, 5 ou 6, caractérisé en ce que la biomasse de la culture préalable ajoutée représente de 10 à 20 % de la biomasse totale.

8. Procédé selon l'une des revendications 4 à 7, caractérisé en ce que les cycles de production avec adjonction d'une culture préalable apte à la croissance suivent les cycles de production sans adjonction de la culture préalable.

9. Procédé selon la revendication 8, caractérisé en ce que l'adjonction de la culture préalable apte à la croissance à l'un des cycles de production consécutifs est réalisée à intervalles de 3 à 5 cycles.

10. Procédé selon l'une des revendications 1 à 9, caractérisé en ce que l'on utilise en tant que microorganisme ayant une activité de deshydrogénase Gluconobacter oxydans.

11. Procédé de préparation de la dihydroacétone par deshydrogénation du glycérol à l'aide de microorganismes ayant une deshydrogénase, cultivés au préalable en l'absence de glycérol et en présence de monosaccharides ou d'alcools de sucres, en plusieurs cycles de production, dans lequel la biomasse obtenue après un cycle de production et qui n'est plus apte à la croissance, est réutilisée en totalité pour le cycle consécutif, caractérisé en ce quel e cycle suivant un cycle de production est réalisé sans adjonction de compléments du milieu de croissance, en présence d'une solution aqueuse contenant des ions calcium, à un pH de 4,0 à 5,5.
